# EUROPÄISCHE PATENTSCHRIFT

(11) **EP 1 796 599 B1**
(45) Veröffentlichungstag und Bekanntmachung des Hinweises auf die Patenterteilung: **10.09.2008**
(21) Anmeldenummer: 04761868.1
(22) Anmeldetag: 13.08.2004
(51) Int. Cl.: A61F 2/44, A61B 17/70

(54) **INTERSPINALES IMPLANTAT**
INTERVERTEBRAL IMPLANT
IMPLANT INTERVERTEBRAL

(43) Veröffentlichungstag der Anmeldung: 20.06.2007
(73) Patentinhaber: Synthes GmbH, 4436 Oberdorf (CH)
(72) Erfinder: HARTMANN, Stephan, CH-4500 Solothurn (CH); STUDER, Armin, CH-6330 Cham (CH)
(74) Vertreter: Lusuardi, Werther
(86) Internationale Anmeldenummer: PCT/CH2004/000507
(87) Internationale Veröffentlichungsnummer: WO 2006/015500

(56) Entgegenhaltungen:
- WO-A-02/03882
- FR-A- 2 717 675

## Beschreibung

Die Erfindung bezieht sich auf ein interspinales Implantat gemäss dem Oberbegriff des Patentanspruchs 1.

Solche interspinale Implantate dienen als Abstandhalter für zwei benachbarte Wirbelkörper bei einer defekten Bandscheibe, welche den Abstand zwischen den Wirbelkörpern sonst verringern würden. Durch den wieder vergrösserten Abstand werden auch die Fazetten-Gelenke entlastet.

Ein gattungsgemässes interspinales Implantat ist aus der FR 2 717 675 TAYLOR bekannt, die den nächsten Stand der Technik darstellt. Dieses bekannte Implantat umfasst zwei an den Dornfortsätzen der Wirbelkörper befestigbar starre Einsatzstücke und dazwischen ein elastisches Mittelstück. Die Einsatzstücke weisen an ihren Grundflächen widerhakenartige Ansätze auf, welche im elastischen Mittelstück eingegossen sind und die Einsatzstücke mit dem Mittelstück zusammenhalten. Nachteilig an diesem bekannten Implantat ist, dass bei der Implantation das bereits komplett vormontierte Implantat zwischen die Dornfortsätze eingeführt wird, so dass die angrenzenden Wirbelkörper unter Umständen weit distrahiert werden müssen.

Ferner ist aus der WO 03/015645 MATHIEU eine gattungsgemässe interspinale Prothese bekannt. Diese bekannte interspinale Prothese umfasst zwei Teile mit Fortsätzen zur Anlage an die Dornfortsätze der zu behandelnden Wirbelkörper und ein fest mit den Fortsätzen eines Teiles verbundenes Mittelteil, so dass jedes Teil lateral, d.h. von einer anderen Seite der Wirbelsäule her eingeführt werden muss.

Hier will die Erfindung Abhilfe schaffen. Der Erfindung liegt die Aufgabe zugrunde, ein interspinales Implantat zu schaffen, welches monolateral in den Interspinalraum zwischen zwei Dornfortsätzen zweier zu behandelnder Wirbelkörper einführbar und fixierbar ist.

Die Erfindung löst die gestellte Aufgabe mit einem interspinalen Implantat, welches die Merkmale des Anspruchs 1 aufweist.

Die durch die Erfindung erreichten Vorteile sind im wesentlichen darin zu sehen, dass dank des erfindungsgemässen Implantates:
- eine Implantation mit einem monolateralen Zugang ermöglicht wird und damit der Operationsvorgang weniger invasiv ist;
- eine direkte Distraktion der beiden angrenzenden Wirbelköper mit dem Implantat möglich ist, was eine bessere Kontrolle ermöglicht; und
- wegen der beiden vorangehend genannten Vorteilen eine kürzere Operationsdauer möglich ist.

Bei einer besonderen Ausführungsform sind die ersten und zweiten Strukturen parallel zu einer die ventrale und die dorsale Seitenflächen schneidenden, quer zur Zentralachse stehenden Querachse formschlüssig ausgebildet.
Die ersten und zweiten Strukturen können parallel zur Zentralachse formschlüssig ausgebildet sein.
Bei einer weiteren Ausführungsform umfasst das interspinale Implantat Arretiermittel, mittels welcher die ersten und zweiten Strukturen quer zur Zentralachse im Eingriff haltbar sind. Dadurch ist das Mittelteil gegen ein Herausrutschen gesichert. Durch das automatische Arretieren ist keine Operation nötig.
Die Arretiermittel können lösbar ausgebildet sein.

Bei einer weiteren Ausführungsform umfassen die ersten Strukturen je mindestens eine männliche Schalbenschwanzführung und die zweiten Strukturen je mindestens eine passende weibliche Schwalbenschwanzführung mit einer quer zur Zentralachse verlaufenden Längsachse umfassen. Diese Schwalbenschwanzführungen gestatten ein spielfreies Zusammenfügen der drei Teile, so dass nach der Implantation die Bewegungen der angrenzenden Wirbelkörper durch das elastische Mittelteil gedämpft werden.
Die männlichen und weiblichen Schwalbenschwanzführungen können quer zur Querachse verlaufende Längsachsen (26) aufweisen. Dadurch ist ein monolaterales Einführen der Einsatzteile sowie des Mittelteils gewährleistet.

Bei einer weiteren Ausführungsform umfassen die Arretiermittel in die Einsatzteile (3;4) einschraubbare Schrauben, mittels welcher die männlichen Schwalbenschwanzführung in den weiblichen Schwalbenschwanzführungen gegen Verschiebungen blockierbar sind. Der Vorteil dieser Ausführung liegt in der lösbaren Ausgestaltung der Arretiermittel.

Bei einer weiteren Ausführungsform sind die weiblichen Schwalbenschwanzführungen gegen diejenigen lateralen Seitenflächen geschlossen, welche keine Verbindungsmittel aufweisen. Dadurch muss pro Schwalbenschwanzführung als Arretiermittel nur eine Schraube eingedreht werden.

Bei einer weiteren Ausführungsform verjüngen sich die männlichen und weiblichen Schwalbenschwanzführungen gegen diejenigen lateralen Seitenflächen des Implantates, welche keine Verbindungsmittel aufweisen. Das Einführen der männlichen Schwalbenschwanzführungen in die weiblichen Schwalbenschwanzführungen wird dadurch erleichtert.
Die Längsachse der Schwalbenschwanzführungen kann gekrümmt sein. Die Krümmung der Schwalbenschwanzführungen ermöglicht ein Einführen des Mittelteils mittels eines schwenkbaren Spannhebels, wodurch der Raumbedarf während des Einführens minimal gehalten werden kann.
Alternativ kann Längsachse der Schwalbenschwanzführungen auch geradlinig sein. Der Vorteil dieser Ausführung liegt in der einfachen Herstellung der Schwalbenschwanzführungen.

Bei einer weiteren Ausführungsform ist das interspinale Implantat elastisch deformierbar. Durch den modularen Aufbau des interspinalen Implantates sind Mittelteile mit verschiedenen Höhen und Steifigkeiten einsetzbar, so dass das interspinale Implantat optimal an die Anatomie anpassbar ist.

Bei einer weiteren Ausführungsform_weist das interspinale Implantat eine progressive Federkennlinie auf. Die progressive Federkennlinie gewährleistet bei kleinen Federwegen eine ausreichend grosse elastische Nachgiebigkeit. Zudem lässt sich auch bei Stossbelastungen eine Überlastung der Wirbelkörper oder der im Zwischenwirbelraum liegende Bandscheibe verhindern.

Die Arretiermittel können selbsttätig einrastbar ausgebildet sind, was ein einfaches Zusammenfügen des Implantates gestattet. Bei jeder weiblichen Schwalbenschwanzführung kann eine Vertiefung und an jeder männlichen Schwalbenschwanzführung ein in die entsprechende Vertiefung einrastbares Federelement (30) vorgesehen sein, so dass die Arretiermittel eine besonders einfache Ausgestaltung aufweisen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Ausführungsform des erfindungsgemässen interspinalen Implantates;
Fig. 2 eine perspektivische Ansicht des Mittelteiles zusammen mit dem zweiten Einsatzteil der in Fig. 1 dargestellten Ausführungsform des erfindungsgemässen interspinalen Implantates;
Fig. 3 eine perspektivische Ansicht einer Ausführungsform des erfindungsgemässen interspinalen Implantates;
Fig. 4 eine Ansicht auf die in Fig. 3 dargestellte Ausführungsform des erfindungsgemässen interspinalen Implantates; und
Fig. 5 eine Draufsicht auf die in den Fig. 3 und 4 dargestellten Ausführungsform des erfindungsgemässen interspinalen Implantates;
Fig. 6 einen vergrösserten Ausschnitt gemäss dem Kreis A in Fig. 4.
Fig. 7 eine perspektivische Ansicht der zwei Einsatzteile einer Ausführungsform des erfindungsgemässen interspinalen Implantates während der Implantation;
Fig. 8 eine perspektivische Ansicht einer Ausführungsform des erfindungsgemässen interspinalen Implantates während des Einschiebens des Mittelteils;
Fig. 9 eine perspektivische Ansicht einer Ausführungsform des Instrumentes; und
Fig. 10 einen Vergrösserung des Kreises II in Fig. 9.

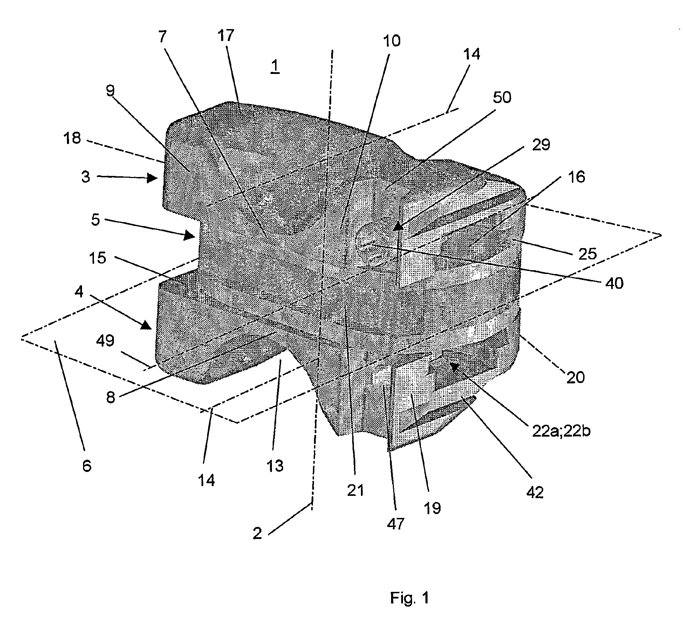

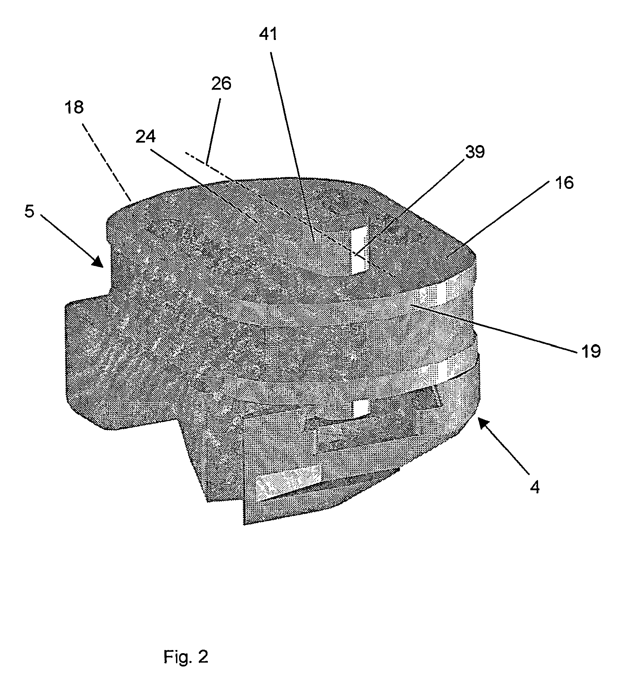

Das in den Fig. 1 und 2 dargestellte interspinale Implantat 1 umfasst im wesentlichen zwei quer zur Zentralachse 2 des interspinalen Implantates 1 angeordnete, U-förmige Einsatzteile 3;4 und ein dazwischen angeordnetes Mittelteil 5. Die Einsatzteile 3;4 sind bezüglich einer zur Längsachse 2 orthogonalen Mittelebene 6 derart angeordnet, dass die Querstege 7 der zwei Einsatzteile 3;4 parallel zur Mittelebene 6 angeordnet sind und die freien Enden 11;12 der Seitenstege 9;10 von der Mittelebene 6 weggerichtet sind.

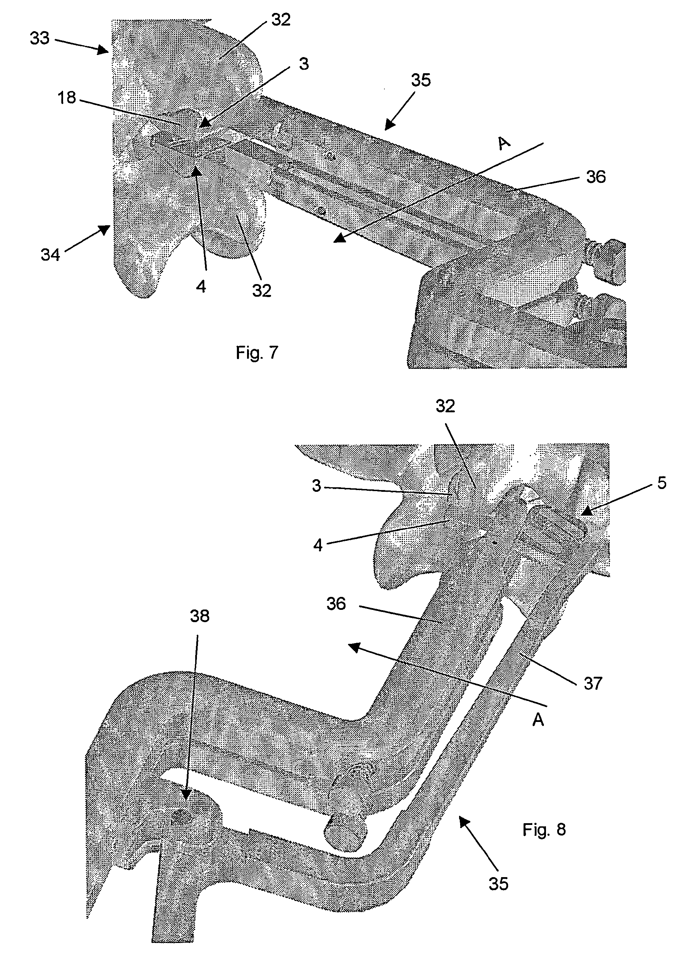

Je ein durch zwei Seitenstege 9;10 und einen Quersteg 7;8 eines Einsatzteiles 3;4 eingegrenzter Kanal 13 ist zur Aufnahme eines Dornfortsatzes eines Wirbelkörpers geeignet, wobei die Einsatzteile 3;4 derart in den interspinalen Zwischenraum eingesetzt werden, dass die Querstege 7;8 zwischen den Dornfortsätzen der zwei aneinander grenzenden Wirbelkörper angeordnet sind. Die Kanalachsen 14 der Kanäle 13 stehen quer zur Zentralachse 2 in antero-posteriorer Richtung, d.h sie verlaufen parallel zu einer die ventrale und die dorsale Seitenfläche 20;21 schneidenden Querachse 49 des interspinalen Implantates 1. Das Mittelteil 5 ist zwischen den gegeneinander gerichteten Grundflächen 15 der Querstege 7 eingesetzt, wobei die Auflageflächen 16 des Mittelteils 5 an den Grundflächen 15 der Querstege 7 zur Anlage kommen. Die Einsatzteile 3;4 umfassen aussen an den Seitenstegen 9;10 liegende laterale Seitenflächen 18;19, quer dazu je eine ventrale Seitenfläche 20 und je eine dorsale Seitenfläche 21 sowie je eine einen Kanal 13 aufweisenden Appositionsfläche 17. Zur Aufnahme der Greifelemente 43 eines chirurgischen Instrumentes 35 (Fig. 7 bis 10) sind an den zweiten lateralen Seitenflächen 19 Verbindungsmittel angebracht.

An jeder Auflageflächen 16 des Mittelteils 5 und jeder Grundfläche 15 der Einsatzteile 3;4 sind die ersten und zweiten Strukturen 22a;22b angeordnet. Die ersten und zweiten Strukturen 22a;22b sind als Schwalbenschwanzführungen mit je einer männlichen Schwalbenschwanzführung 24 an den Auflageflächen 16 des Mittelteils (Fig. 2) und einer weiblichen Schwalbenschwanzführung 25 an der Grundfläche 15 der Einsatzteile 3;4 ausgestaltet und sind dazu geeignet, die Einsatzteile 3;4 mit dem Mittelteil 5 lösbar zu verbinden. Die männlichen und weiblichen Schwalbenschwanzführungen 24;25 weisen eine quer zur Zentralachse 2 angeordnete und in der Mittelebene 6 liegende Längsachse 26 (Fig. 2) auf. Ferner sind die Schwalbenschwanzführungen 24;25 derart ausgerichtet, dass die Längsachse 26 (Fig. 2) quer zu den Kanalachsen 14 verläuft. Die weiblichen Schwalbenschwanzführungen 25 sind gegen die ersten lateralen Seitenflächen 18 der Einsatzteile 3;4 geschlossen und gegen die zweiten lateralen Seitenflächen 19 offen, so dass die männlichen Schwalbenschwanzführungen 24 von den zweiten lateralen Seitenflächen 19 der Einsatzteile 3;4 her in die weiblichen Schwalbenschwanzführungen 25 eingeführt werden können. Die männlichen Schwalbenschwanzführungen 24 sind kürzer als die weiblichen Schwalbenschwanzführungen 25, so dass bei zwischen den Einsatzteilen 3;4 eingefügtem Mittelteil 5 zwischen dem eine Erhebung 41 aufweisenden hinteren Ende 39 (Fig. 2) der männlichen Schwalbenschwanzführung 24 und der zweiten lateralen Seitenfläche 19 der beiden Einsatzteile 3;4 je eine Schraube 40 in die Bohrungen 47 in den Seitenstegen 10 einschraubbar ist. Die als Arretiermittel 29 dienende Schraube 40 verschliesst die weibliche Schwalbenschwanzführung 25 derart, dass die Erhöhung 41 am hinteren Ende 39 der männlichen Schwalbenschwanzführung 24 an der Schraube 40 anliegt. Durch die in den geschlossenen weiblichen Schwalbenschwanzführungen 25 mittels der Schrauben 40 gegen Verschiebungen in beiden Richtungen gesicherten männlichen Schwalbenschwanzführungen 24 werden die zwei Einsatzteile 3;4 nach dem Eindrehen der zwei Schrauben 40 relativ zum Mittelteil 5 blockiert. Die Seitenstege 10 sind mit Vertiefungen 50 ausgebildet, so dass der Kopf der Schrauben 40 in den Vertiefungen 50 versenkt ist und nicht über die dorsalen Seitenflächen 21 der Einsatzteile 3;4 vorsteht.

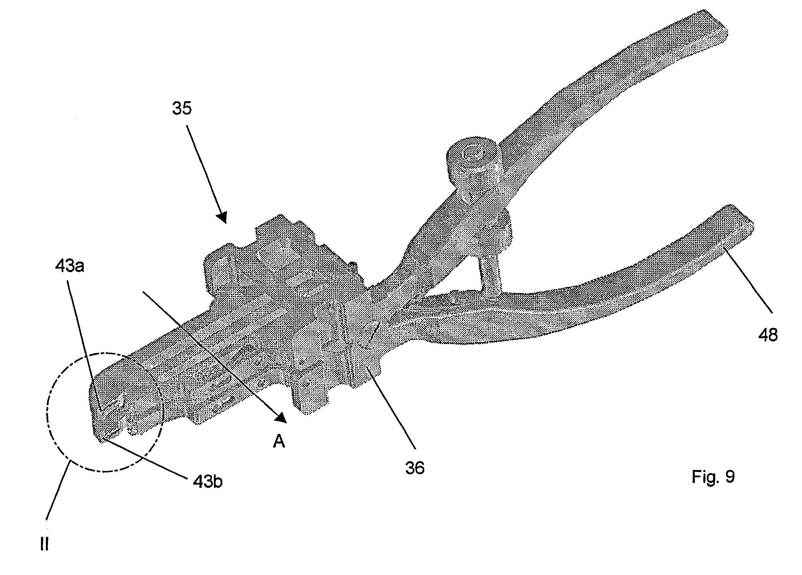

An den lateralen Seitenflächen 18;19 der zwei Einsatzteile 3;4 sind zu den Kanalachsen 14 parallele Nuten 42 angeordnet. Die Nuten 42 sind an den dorsalen Seitenflächen 21 offen und an den ventralen Seitenflächen 20 geschlossen und dienen zur Aufnahme der Greifelemente 43 eines chirurgischen Instrumentes 35 (Fig. 9).

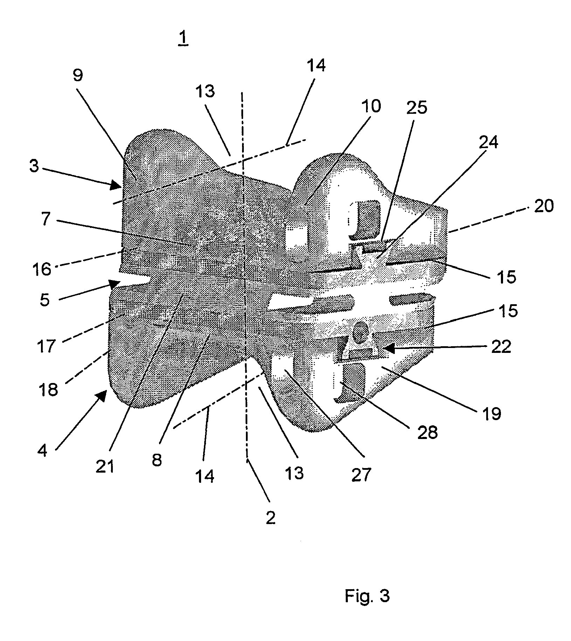

Die in den Fig. 3 bis 5 dargestellte Ausführungsform des interspinalen Implantates 1 unterscheidet sich von der in den Fig. 1 und 2 dargestellten Ausführungsform darin, dass die Arretiermittel 29, die männlichen und weiblichen Schwalbenschwanzführungen 24;25 sowie die Aufnahmen für die Greifelemente eines chirurgischen Instrumentes 35 (Fig. 7) anders ausgestaltet sind. Die Arretiermittel 29 umfassen im wesentlichen ein in einer Vertiefung 31 einrastbares Federelement 30, welches in der hier dargestellten Ausführungsform als am Mittelteil 5 befestigte Blattfeder ausgebildet ist. Die Vertiefung 31 ist an der weiblichen Schwalbenschwanzführung 25 angeordnet, und weist parallel zur Längsachse 26 der Schwalbenschwanzführungen 24;25 eine Länge L auf, welche derart bemessen ist, dass das Feldelement 30 dann in die Vertiefung 31 einrastet, wenn das Mittelteil 5 beim Einschieben zwischen die Einsatzeile 3;4 quer zur Zentralachse 2 seine endgültige Position relativ zu den Einsatzteilen 3;4 erreicht hat. In der hier dargestellten Ausführungsform wird das Mittelteil 5 durch die geschlossenen weiblichen Schwalbenschwanzführungen 25 in Richtung der ersten lateralen Seitenflächen 18 und durch das Federelement 30 in Richtung der zweiten lateralen Seitenflächen 19 in seiner Position gehalten.

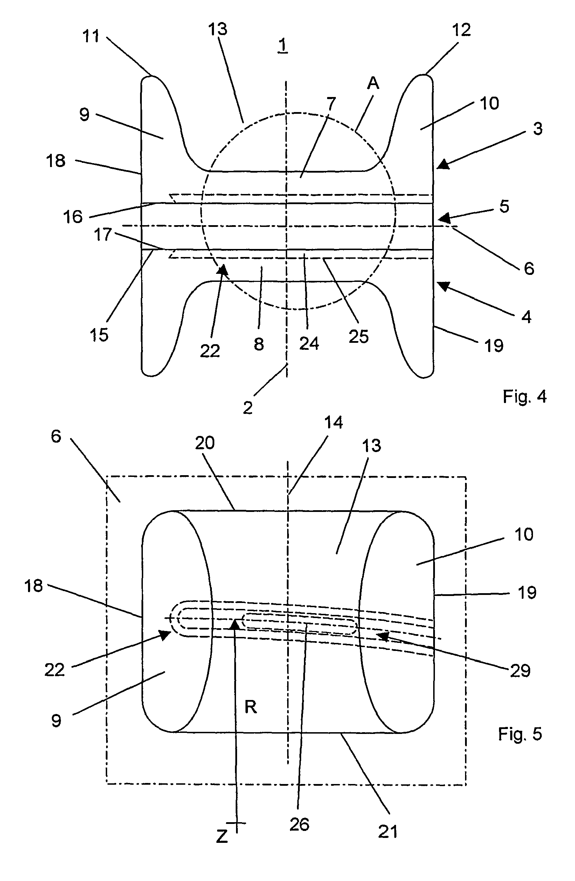

Die Schwalbenschwanzführungen 24 sind derart ausgebildet, dass ihre Längsachse 26 (Fig. 5) gekrümmt ist. Damit die Einsatzteile 3;4 nach der Implantation des interspinalen Implantates 1 auch parallel zu den Grundflächen 15 relativ zum Mittelteil 5 fixiert sind, sind zwischen jeder der zwei männlichen Schwalbenschwanzführung 24 und jeder der zwei weiblichen Schwalbenschwanzführungen 25 ineinander einrastbare Arretiermittel 29 vorgesehen.

Ferner sind an den zwei Einsatzteilen 3;4 von den dorsalen Seitenflächen 21 her in die Seitenstege 10 eindringende, erste ovale Vertiefungen 27 und von den - von dorsal betrachtet - rechts angeordneten, zweiten Seitenflächen 19 her in die Seitenstege 10 eindringende, zweite ovale Vertiefungen 27 angebracht. In der hier dargestellten Ausführungsform sind die ersten und zweiten ovalen Vertiefungen 27;28 - von dorsal betrachtet - in die rechten Seitenstege 1 der zwei Einsatzteile 3;4 eingelassen. Diese ersten und zweiten ovalen Vertiefungen 27;28 sind zur Aufnahme der Greifelemente eines chirurgischen Instrumentes 35 (Fig. 7) geeignet.

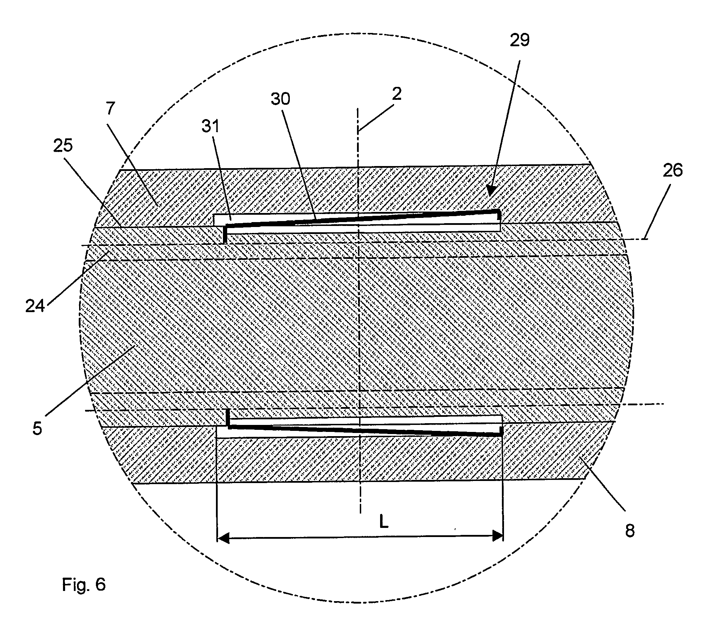

Wie in Fig. 6 dargestellt umfassen die Arretiermittel 29 im wesentlichen ein in einer Vertiefung 31 einrastbares Federelement 30, welches in der hier dargestellten Ausführungsform als am Mittelteil 5 befestigte Blattfeder ausgebildet ist. Die Vertiefung 31 ist an der weiblichen Schwalbenschwanzführung 25 angeordnet, und weist parallel zur Längsachse 26 der Schwalbenschwanzführungen 24;25 eine Länge L auf, welche derart bemessen ist, dass das Federlement 30 dann in die Vertiefung 31 einrastet, wenn das Mittelteil 5 beim Einschieben zwischen die Einsatzeile 3;4 quer zur Zentralachse 2 seine endgültige Position relativ zu den Einsatzteilen 3;4 erreicht hat. In der hier dargestellten Ausführungsform wird das Mittelteil 5 durch die geschlossenen weiblichen Schwalbenschwanzführungen 25 in Richtung der ersten lateralen Seitenflächen 18 und durch das Federelement 30 in Richtung der zweiten lateralen Seitenflächen 19 in seiner Position gehalten.

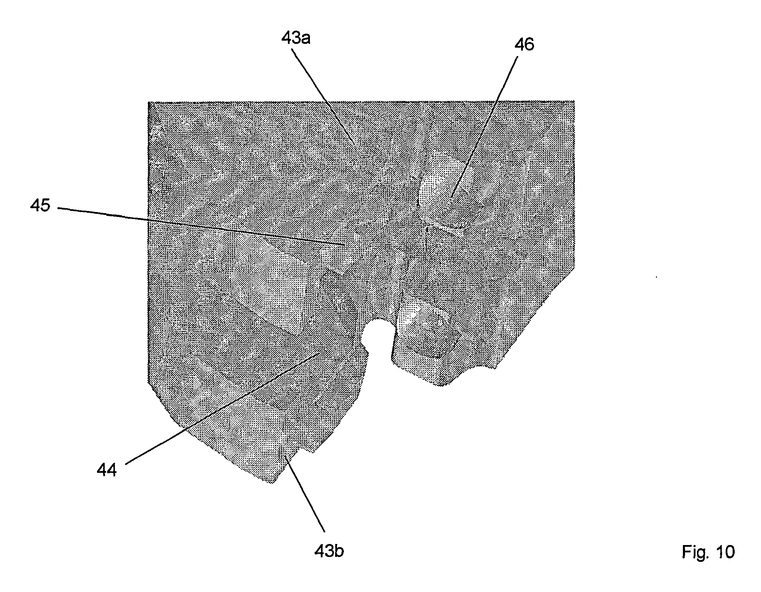

Die Fig. 7 und 8 dienen zur Erläuterung des folgenden Operationsvorganges zur Implantation des interspinalen Implantates 1:
a) Herstellen einer monolateralen Inzision bei den zu behandelnden Wirbelkörpern 33;34;
b) Distrahieren der zu behandelnden Wirbelkörper 33;34 mittels einer durch die laterale Inzision durchführbaren, an den Dornfortsätzen 32 der zwei Wirbelkörper 33;34 zur Anlage bringbaren Zange (nicht gezeichnet);
c) Einführung der zwei Einsatzteile 3;4 mittels einer Haltezange 36 (Fig. 7 bis 10) durch die laterale Inzision, wobei in der hier dargestellten Ausführungsform der interspinalen Prothese die Einsatzteile 3;4 von rechts in Richtung des Pfeils A in die Zwischenräume zwischen den Dornfortsätzen 32 der zwei zu behandelnden Wirbelkörper 33;34 eingeführt werden. Die freien Enden der Haltezange 36 werden dazu in den ovalen Vertiefungen 27;28 (Fig. 3) oder in den Nuten 42 (Fig. 1) verankert, so dass die zwei Einsatzteile 3;4 mit ihren ersten lateralen Seitenflächen 18 voraus in den Zwischenraum der Dornfortsätze 32 geschoben werden können;
d) Einführung des Mittelteils 5 von lateral zwischen die zwei Einsatzteile 3;4 mittels des Spannhebels 37 (Fig. 7 und 8) oder durch den Zuführkanal 44 (Fig. 9 und 10);
e) Erzeugen einer Vorspannung auf das Mittelteil 5 durch leichtes Distrahieren des zwei Einsatzteile 3;4 mittels der Haltezange 36;
f) Lösen der Fixierung der freien Enden der Haltezange 36 von den zwei Einsatzteilen 3;4;
g) Entfernen der Haltezange 36; und
h) Schliessen der Inzision.

In den Fig. 9 und 10 ist eine Ausführungsform des chirurgischen Instrumentes 35 dargestellt, welche für die in den Fig. 1 und 2 dargestellte Ausführungsform des Interspinalen Implantates 1 geeignet ist. Die Greifelemente 43a;43b sind an ihren freien Enden mit Erhebungen 45 versehen, welche mit den Nuten 42 an den lateralen Seitenflächen 18; 19 der Einsatzteile 3;4 in Eingriff bringbar sind.

Die Schrauben 40 werden nach der Ausführung des Verfahrensschrittes d) durch die Durchgangsöffnungen 46 parallel zu den Kanalachsen 14 durchgeschoben und in die Seitenstege 10 der Einsatzteile 3;4 eingeschraubt.

In der in den Fig. 7 und 8 dargestellten Ausführungsform des chirurgischen Instrumentes 35 wird das Mittelteil 5 gemäss Verfahrensschritt d) nicht durch einen Zuführkanal 44 sonder mittels eines Spannhebels 37 zwischen die Einsatzteile 3;4 geschoben, so dass nach dem Verfahrensschritt c) folgende Verfahrensschritte ausgeführt werden müssen:
d') Einführung eines Spannhebels 36 durch die laterale Inzision, wobei am vorderen Ende des Spannhebels 37 das Mittelteil 5 eingespannt ist;
d") Einführung des Mittelteils 5 von lateral zwischen die zwei Einsatzteile 3;4 mittels des Spannhebels 37. Zur einfacheren Durchführung dieses Schrittes kann der Spannhebel 37 mittels eines Scharniers 38 (Fig. 8) an der Haltezange 36 drehbar gelagert sein;
e) Erzeugen einer Vorspannung auf das Mittelteil 5 durch leichtes Distrahieren des zwei Einsatzteile 3;4 mittels der Haltezange 36;
f) Entfernen des Spannhebels 37;
g) Lösen der Fixierung der freien Enden der Haltezange 36 von den zwei Einsatzteilen 3;4;
h) Entfernen der Haltezange 36; und
i) Schliessen der Inzision.

## Patentansprüche

1. Interspinales Implantat (1) mit einer Zentralachse (2) umfassend
A) zwei bezüglich der Zentralachse (2) axial übereinander angeordnete, U-förmige Einsatzteile (3;4) mit gegeneinander gerichteten Grundflächen (15), je einer ersten und einer zweiten lateralen Seitenfläche (18;19), je einer ventralen und einer dorsalen Seitenfläche (20;21) und je einer einen Kanal (13) zur Aufnahme der Dornfortsätze (32) zweier benachbarter Wirbelkörper (33;34) aufweisenden Appositionsfläche (17);
B) ein quer zur Zentralachse (2) zwischen die Grundflächen (15) einführbares Mittelteil (5), wobei
C) das Mittelteil (5) zwei zur Anlage an die Grundflächen (15) geeignete Auflageflächen (16) aufweist, und jede der Auflageflächen mit einer ersten dreidimensionalen Struktur (22a) versehen ist; und
D) jede der Grundflächen (15) eine mit der ersten dreidimensionalen Struktur (22a) in Eingriff bringbare, zweite dreidimensionale Struktur (22b) aufweist, so dass die Einsatzteile (3;4) bei in Eingriff stehenden ersten und zweiten Strukturen (22a;22b) am Mittelteil (5) parallel zur Zentralachse (2) lösbar fixiert sind,
**dadurch gekennzeichnet, dass**
E) die Einsatzteile (3;4) je auf derselben lateralen Seitenfläche (18;19) Verbindungsmittel (23) zur Befestigung der Einsatzteile (3;4) an den Greifelementen (43) eines chirurgischen Instrumentes (35) aufweist; und
F) die ersten und zweiten Strukturen (22a;22b) nur von der die Verbindungsmittel aufweisenden lateralen Seitenfläche (18;19) in Eingriff, respektive ausser Eingriff bringbar sind.

2. Interspinales Implantat (1) nach Anspruch 1, **dadurch gekennzeichnet, dass** die ersten und zweiten Strukturen (22a;22b) parallel zu einer die ventrale und die dorsale Seitenflächen (20;21) schneidenden, quer zur Zentralachse (2) stehenden Querachse (49) formschlüssig ausgebildet sind.

3. Interspinales Implantat (1) nach 1 oder 2, **dadurch gekennzeichnet, dass** die ersten und zweiten Strukturen (22a;22b) parallel zur Zentralachse (2) formschlüssig ausgebildet sind.

4. Interspinales Implantat (1) nach einem der Ansprüche 1 bis 3, **dadurch gekennzeichnet, dass** sie Arretiermittel (29) umfasst, mittels welcher die ersten und zweiten Strukturen (22a;22b) quer zur Zentralachse (2) im Eingriff haltbar sind.

5. Interspinales Implantat (1) nach Anspruch 4, **dadurch gekennzeichnet, dass** die Arretiermittel (29) lösbar sind.

6. Interspinales Implantat (1) nach einem der Ansprüche 1 bis 5, **dadurch gekennzeichnet, dass** die ersten Strukturen (22a) je mindestens eine männliche Schalbenschwanzführung (24) und die zweiten Strukturen (22b) je mindestens eine passende weibliche Schwalbenschwanzführung (25) mit einer quer zur Zentralachse (2) verlaufenden Längsachse (26) umfassen.

7. Interspinales Implantat (1) nach Anspruch 6, **dadurch gekennzeichnet, dass** die männlichen und weiblichen Schwalbenschwanzführungen (24;25) quer zur Querachse (49) verlaufende Längsachsen (26) aufweisen.

8. Interspinales Implantat (1) nach Anspruch 6 oder 7, **dadurch gekennzeichnet, dass** die Arretiermittel (29) in die Einsatzteile (3;4) einschraubbare Schrauben (40) umfassen, mittels welcher die männlichen Schwalbenschwanzführungen (24) in den weiblichen Schwalbenschwanzführungen (25) gegen Verschiebungen blockierbar sind.

9. Interspinales Implantat (1) nach einem der Ansprüche 6 bis 8, **dadurch gekennzeichnet, dass** die weiblichen Schwalbenschwanzführungen (25) gegen diejenigen lateralen Seitenflächen (18;19) geschlossen sind, welche keine Verbindungsmittel aufweisen.

10. Interspinales Implantat (1) nach einem der Ansprüche 6 bis 9, **dadurch gekennzeichnet, dass** sich die männlichen und weiblichen Schwalbenschwanzführungen (24;25) gegen diejenigen lateralen Seitenflächen (18;19) des Implantates (1) verjüngen, welche keine Verbindungsmittel aufweisen.

11. Interspinales Implantat (1) nach einem der Ansprüche 6 bis 10, **dadurch gekennzeichnet, dass** die Längsachse (26) der Schwalbenschwanzführungen (24;25) gekrümmt ist.

12. Interspinales Implantat (1) nach einem der Ansprüche 6 bis 10, **dadurch gekennzeichnet, dass** die Längsachse (26) der Schwalbenschwanzführungen (24;25) geradlinig ist.

13. Interspinales Implantat (1) nach einem der Ansprüche 1 bis 12, **dadurch gekennzeichnet, dass** es elastisch deformierbar ist.

14. Interspinales Implantat (1) nach Anspruch 13, **dadurch gekennzeichnet, dass** es eine progressive Federkennlinie aufweist.

15. Interspinales Implantat (1) nach einem der Ansprüche 4 bis 7 oder 9 bis 14, **dadurch gekennzeichnet, dass** die Arretiermittel (29) selbsttätig einrastbar ausgebildet sind.

16. Interspinales Implantat (1) nach Anspruch 15, **dadurch gekennzeichnet, dass** an jeder weiblichen Schwalbenschwanzführung (25) eine Vertiefung (31) und an jeder männlichen Schwalbenschwanzführung (24) ein in die entsprechende Vertiefung (31) einrastbares Federelement (30) vorgesehen sind.

## Claims

1. Inter-spinal implant (1) with a central axis (2) comprising
A) two U-shaped inserts (3;4) set over each other with respect to the central axis (2) and with opposing base surfaces (15), each with a first and a second lateral side surface (18;19), a ventral and dorsal side surface (20:21) and a juxtaposition surface (17) having a channel (13) to receive the spinal processes (32) of two adjacent vertebral bodies (33;34);
B) a centre piece (5) introducible across the central axis (2) between the base surfaces (15), where
C) the centre piece (5) presents contact surfaces (16) suitable for matching with the base surfaces (15), and each of the contact surfaces is provided with a first tri-dimensional structure (22a); and
D) each of the base surfaces (15) presents a second tri-dimensional structure (22b) which can be made to engage with the first tri-dimensional structure (22a) so that the inserts (3;4) are, after engaging the first and second structures (22a;22b), fastened in a detachable manner to the centre piece (5) parallel to the central axis (2),
**characterized in that**
E) the inserts (3;4) each present, on the same lateral side surface (18;19), connecting means to fasten the inserts (3;4) to the gripping elements (43) of a surgical instrument (35); and
F) the first and second structures (22a;22b) can be engaged or disengaged only by the lateral side surface (18;19) fitted with the connecting means (23).

2. Inter-spinal implant (1) according to claim 1, **characterized in that** the first and second structures (22a;22b) are conformed in a form-locking manner parallel to a transversal axis (49) set across the central axis (2) and crossing the ventral and dorsal side surfaces (20;21).

3. Inter-spinal implant (1) according to claim 1 or 2, **characterized in that** the first and second structures (22a;22b) are conformed in a form-locking manner parallel to the central axis (2).

4. Inter-spinal implant (1) according to one of the claims from 1 to 3, **characterized in that** it comprises locking means (29) by which the first and second structures (22a;22b) can be held engaged across the central axis (2).

5. Inter-spinal implant (1) according to claim 4, **characterized in that** the locking means (29) are detachable.

6. Inter-spinal implant (1) according to one of the claims from 1 to 5, **characterized in that** the first and structures (22a) each comprise at least one male dove tail guide (24) and the second structures (22b) each comprise at least one suitable dove tail guide (25) with one longitudinal axis (26) running across the central axis (2).

7. Inter-spinal implant (1) according to claim 6, **characterized in that** the male and female dove tail guides (24;25) have longitudinal axes (26) running across the transversal axis (49).

8. Inter-spinal implant (1) according to claim 6 or 7, **characterized in that** the locking means (29) comprise screws (40) that can be screwed into the inserts (3;4), whereby the male dove tail guides (24) can be blocked toward shifting in the female dove tail guides (25).

9. Inter-spinal implant (1) according to one of the claims from 6 to 8, **characterized in that** the female dove tail guides (25) are closed toward the lateral side surfaces (18;19) that do not have connecting means.

10. Inter-spinal implant (1) according to one of the claims from 6 to 9, **characterized in that** the male and the female dove tail guides (24;25) are tapered toward the lateral side surfaces (18;19) of the implant (1) that do not have connecting means.

11. Inter-spinal implant (1) according to one of the claims from 6 to 10, **characterized in that** the longitudinal axis (26) of the dove tail guides (24;25) is curved.

12. Inter-spinal implant (1) according to one of the claims from 6 to 10, **characterized in that** the longitudinal axis (26) of the dove tail guides (24;25) are straight.

13. Inter-spinal implant (1) according to one of the claims from 1 to 12, **characterized in that** it is elastically deformable.

14. Inter-spinal implant (1) according to claim 13, **characterized in that** it presents a progressive characteristic curve of the spring.

15. Inter-spinal implant (1) according to one of the claims from 4 to 7 or from 9 to 14, **characterized in that** the locking means (29) are conformed in a self-acting snap-in manner.

16. Inter-spinal implant (1) according to claim 15, **characterized in that** a recess (31) is provided on each female dove tail guide (25), and that a spring element (30), which is capable of being snapped into the relative recess (31), is provided on each male dove tail guide (24).

## Revendications

1. Implant intervertébral (1) ayant un axe central (2), comprenant
A) deux pièces d'insertion en forme de U (3 ; 4), superposées axialement par rapport à l'axe central (2), présentant des surfaces de base opposées (15), chacune une première et une deuxième faces latérales sur les côtés (18 ; 19), chacune une face latérale ventrale et une face latérale dorsale (20 ; 21) et chacune une surface d'apposition (17) présentant un canal (13) destiné à loger les apophyses épineuses (32) de deux corps vertébraux adjacents (33 ; 34) ;
B) un élément central (5) pouvant être inséré transversalement à l'axe central (2) entre les surfaces de base (15), dans lequel
C) l'élément central (5) présente deux surfaces d'appui (16) appropriées pour l'appui contre les surfaces de base (15), et chacune des surfaces d'appui est pourvue d'une première structure tridimensionnelle (22a) ; et
D) chacune des surfaces de base (15) présente une deuxième structure tridimensionnelle (22b) pouvant être mise en prise avec la première structure tridimensionnelle (22a), de sorte que les pièces d'insertion (3 ; 4) sont fixées de manière amovible au niveau de l'élément central (5) parallèlement à l'axe central (2) lorsque les première et deuxième structures (22a ; 22b) sont en prise,
**caractérisé en ce que**
E) les pièces d'insertion (3 ; 4) présentent chacune, sur la même surface latérale sur le côté (18 ; 19), des moyens de liaison (23) pour la fixation des pièces d'insertion (3 ; 4) sur les éléments de préhension (43) d'un instrument chirurgical (35) ; et
F) les première et deuxième structures (22a ; 22b) peuvent être mises en prise ou hors prise uniquement par la surface latérale sur le côté (18 ; 19) qui présente les moyens de liaison.

2. Implant intervertébral (1) selon la revendication 1, **caractérisé en ce que** les première et deuxième structures (22a ; 22b) sont assemblées par emboîtement parallèlement à un axe transversal (49) disposé transversalement à l'axe central (2) coupant les faces latérales ventrale et dorsale (20 ; 21).

3. Implant intervertébral (1) selon la revendication 1 ou 2, **caractérisé en ce que** les première et deuxième structures (22a ; 22b) sont assemblées par emboîtement parallèlement à l'axe central (2).

4. Implant intervertébral (1) selon l'une quelconque des revendications 1 à 3, **caractérisé en ce qu'**il comprend des moyens d'arrêt (29) qui permettent de maintenir en prise les première et deuxième structures (22a ; 22b) transversalement à l'axe central (2).

5. Implant intervertébral (1) selon la revendication 4, **caractérisé en ce que** les moyens d'arrêt (29) sont amovibles.

6. Implant intervertébral (1) selon l'une quelconque des revendications 1 à 5, **caractérisé en ce que** les premières structures (22a) comprennent chacune au moins un guidage en queue d'aronde mâle (24) et les deuxièmes structures (22b) présentent chacune au moins un guidage en queue d'aronde femelle correspondant (25) avec un axe longitudinal (26) s'étendant transversalement à l'axe central (2).

7. Implant intervertébral (1) selon la revendication 6, **caractérisé en ce que** les guidages en queue d'aronde mâle et femelle (24 ; 25) présentent des axes longitudinaux (26) s'étendant transversalement à l'axe transversal (49).

8. Implant intervertébral (1) selon la revendication 6 ou 7, **caractérisé en ce que** les moyens d'arrêt (29) comprennent des vis (40) pouvant être vissées dans les pièces d'insertion (3 ; 4), vis qui permettent de fixer les guidages en queue d'aronde mâles (24) dans les guidages en queue d'aronde femelle (25) pour empêcher tout déplacement.

9. Implant intervertébral (1) selon l'une quelconque des revendications 6 à 8, **caractérisé en ce que** les guidages en queue d'aronde femelles (25) sont fermées au niveau des faces latérales sur les côtés (18 ; 19) qui ne présentent pas de moyens de liaison.

10. Implant intervertébral (1) selon l'une quelconque des revendications 6 à 9, **caractérisé en ce que** les guidages en queue d'aronde mâles et femelles (24 ; 25) rétrécissent vers les faces latérales sur les côtés (18 ; 19) de l'implant (1) qui ne présentent pas de moyens de liaison.

11. Implant intervertébral (1) selon l'une quelconque des revendications 6 à 10, **caractérisé en ce que** l'axe longitudinal (26) des guidages en queue d'aronde (24 ; 25) est courbé.

12. Implant intervertébral (1) selon l'une quelconque des revendications 6 à 10, **caractérisé en ce que** l'axe longitudinal (26) des guidages en queue d'aronde (24 ; 25) est rectiligne.

13. Implant intervertébral (1) selon l'une quelconque des revendications 1 à 12, **caractérisé en ce qu'**il est déformable de manière élastique.

14. Implant intervertébral (1) selon la revendication 13, **caractérisé en ce qu'**il présente une courbe caractéristique de ressort progressive.

15. Implant intervertébral (1) selon l'une quelconque des revendications 4 à 7 ou 9 à 14, **caractérisé en ce que** les moyens d'arrêt (29) sont conçus de manière auto-encliquetable.

16. Implant intervertébral (1) selon la revendication 15, **caractérisé en ce que** chaque guidage en queue d'aronde femelle (25) comporte un évidement (31) et chaque guidage en queue d'aronde mâle (24) comporte un élément à ressort (30) pouvant être encliqueté dans l'évidement (31) correspondant.
